# EUROPEAN PATENT SPECIFICATION

(11) **EP 0 500 952 B1**
(45) Date of publication and mention of the grant of the patent: **03.03.1999**
(21) Application number: 91915607.5
(22) Date of filing: 09.09.1991
(51) Int. Cl.: C07D 233/52, A61K 31/415, A61K 31/505, A01N 43/50, A01N 43/54, C07D 239/08, C07D 243/04, C07D 401/06

(54) **PROCESS FOR PRODUCING NITROGENOUS HETEROCYCLE**
VERFAHREN ZUR HERSTELLUNG EINES STICKSTOFFHALTIGEN HETEROZYKLUS
PROCEDE POUR PRODUIRE UN HETEROCYCLE AZOTE

(30) Priority: 11.09.1990 JP 239099/90
(43) Date of publication of application: 02.09.1992
(73) Proprietor: NIPPON SODA CO., LTD., Chiyoda-ku, Tokyo 100-8165 (JP)
(72) Inventor: KOJIMA, Shigeru, Nihongi Plant Nipp. Soda Co.,Ltd, Niiggata 949-23 (JP); FUNABORA, M., Nihongi Plant Nipp. Soda Co., Ltd., Niigata 949-23 (JP); KAWAHARA, N., Nihongi Plant Nipp. Soda Co., Ltd., Niigata 949-23 (JP); IIYOSHI, Y., Nihongi Plant Nippon Soda Co., Ltd., Niigata 949-23 (JP)
(74) Representative: von Hellfeld, Axel, Dr. Dipl.-Phys.
(86) International application number: PCT/JP91/01197
(87) International publication number: WO 92/04329

(56) References cited:
- EP-A- 192 060
- EP-A- 0 277 317
- JP-A-63 287 764
- CHEMICAL ABSTRACTS, vol. 114, no. 7, 18 February 1991, Columbus, Ohio, US; abstract no. 62097t, SHIOKAWA, KOZO, A.O. 'PREPARATION OF 2-(NITROIMINO)IMIDAZOLIDINE AS INSECTICIDES.' page 691 ;column 1 ;

## Description

The present invention relates to a process for the preparation of nitrogen containing heterocyclic compounds which are useful for pesticides or intermediates of pharmaceuticals and pesticides.

A synthetic method of compounds represented by general formula [III] was presented in "Journal of the American Chemical Society", 70, 430 (1948), but the yield was insufficient.

JP-A-63/287764 discloses the preparation of compounds similar to compounds of formula [III] (cf. infra) by reacting a diamine and nitromethylene- or cyanoimino-dithiocarbonate. However, nitroimido compounds of present formula [III] are prepared by reacting a diamine shown as formula [IV] and nitroguanidine (cf. comparative example 1 of the application).

Purpose of the present invention is to prepare the nitrogen containing heterocyclic compounds more efficiently than so far by using a novel process composed of reactions between esters of N-nitroimidodithiocabonic acids and diamines.

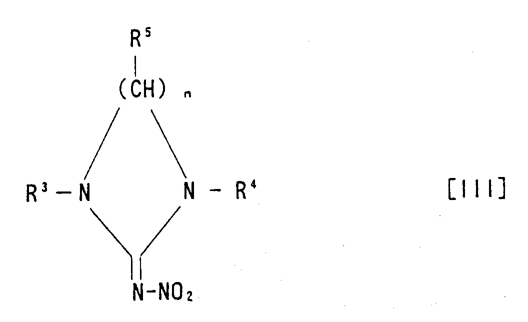

### Disclosure of Invention

The present invention is composed of the following synopses;
synthesizing process of the nitrogen containing heterocycles represented by the general formula [III] (wherein, R³ and R⁴ represent a hydrogen atom, an alkyl group or an alkyl group substituted by heterocycle respectively, R⁵ is a hydrogen atom or an alkyl group and n is 2 to 4), by a reaction between esters of N-nitroimidodithiocabonic acid represented by general formula [I] (wherein, R¹ and R² represent an alkyl group respectively) and diamines represented by general formula [II] (wherein, R³, R⁴, R⁵ and n are the same as defined above)

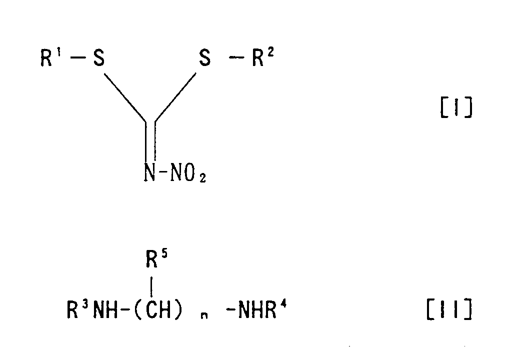

A term of an alkyl group means hereupon a straight or branched chained one having 1 to 4 carbon atoms. For example, methyl, ethyl, isopropyl and tertiary butyl may be given.

A heterocyclic ring which is defined in the expression of " an alkyl group substituted by heterocycle" means nitrogen containing five or six membered ring allowed to be substituted by halogen atom such as chlorine and bromine atom, or alkyl group such as methyl and ethyl group.

For examples of the esters of N-nitroimidodithiocabonic acids represented by the general formula [I] (wherein, R¹ and R² are the same as defined above), S,S'-dimethyl N-nitoroimidodithiocarbonate as R¹ and R² being identical alkyl group, or S-isopropyl S'-methyl N-nitroimidodithiocarbonate and S-methyl S'-t-butyl N-nitroimidodithiocarbonate as different alkyl groups may be given.

Thease compounds can be easily obtained. For example, they can be obtained according to the following equation:

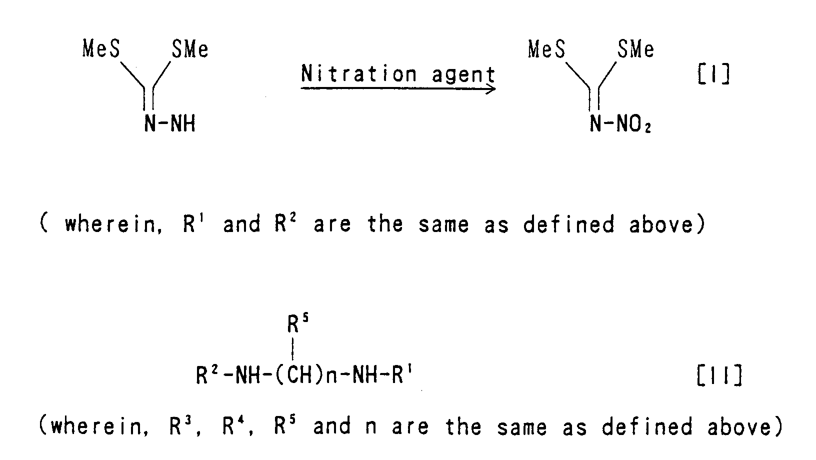

For examples of the diamines represented by the general formula [II], ethylenediamine, N-(2-chloro-5-pyridylmethyl)ethylenediamine, N-(2-chloro-5-thiazoylmethyl)ethylenediamine, N-(2-chloro-5-pyridylmethyl)-N'-methylethylenediamine and the like may be given.

They can be prepared according to methods such as described in " Shin Jikken Kagaku Kouza" vol. 14 (III) pp 1332-1399.

Based on one mole of the esters of N-nitroimidodithiocarbonic acids represented by the general formula [I], 1.0 to 3.0 mole, preferably 1.0 to 1.5 mole of the diamines may be used.

The reaction solvent may not be particularly limited as far as it is inert for reagents and reaction products. Generally, halogenated hydrocarbon such as chloroform or dichloromethane, alcohol such as methanol or ethanol and aromatic hydrocarbon such as toluene or chlorobenzene may be used.

The reaction temperature is from -30 °C to the boiling point of using solvent. Generally, the reaction can be proceed enough at around of the room temperature.

After completion of the reaction, the desired substance with high purity can be obtained by a direct filtration of deposited crystals from the reaction mixture. It is also able to be obtained by using common post-treatment such as recrystallization or column chromatography of residue material after evaporation of the solvents.

When either R³ or R⁴ is hydrogen or both R³ and R⁴ are hydrogen atoms in the general formula [IV], tautomeric isomers exist as follows:

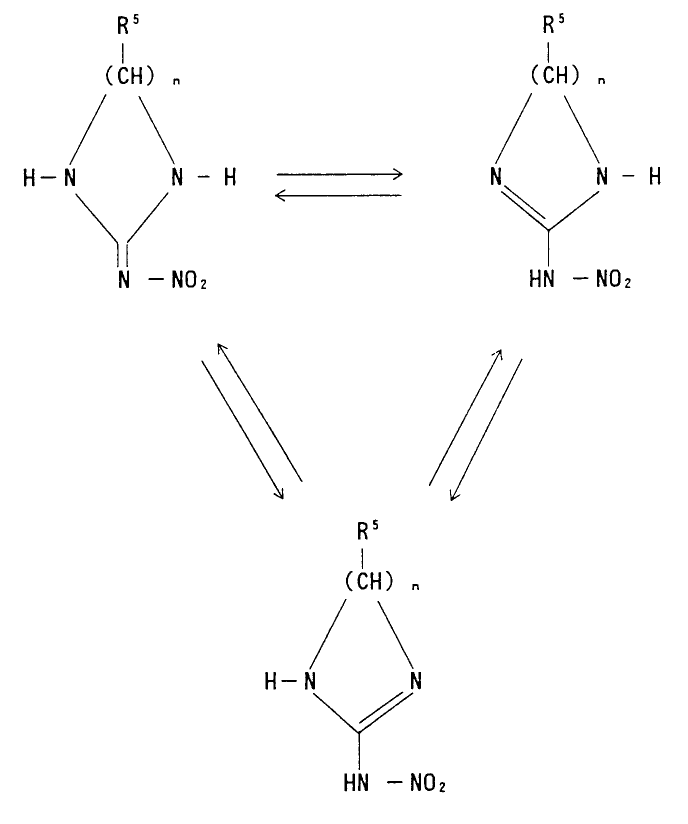

### The Best Mode for Carrying Out the Invention

The present invention is described in more detail with examples, but the invention shall not be limited within the examples.

### (Reference Example)

### Dimethyl N-nitroimidodithiocarbonate:

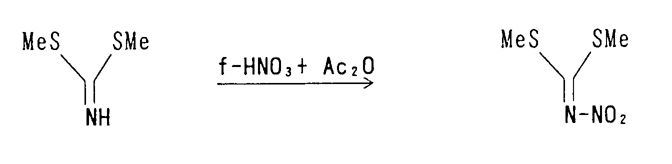

Into 122.4 g (1.2 mol) of acetic acid anhydride, 25.2 g (0.4 mol) of fuming nitric acid (spgr. 1.52) was dropped with stirring below 10 °C over 10 minutes. After cooling to 0°C, 0.3 g of conc. sulfuric acid and 24.2 g (0.2 mol) of dimethyl imidodithiocarbonate was dropped to the mixture at -5 to 5°C for 30 minutes. The reaction mixture was stirred at -5 to 0°C for more 70 minutes, then, the reaction mixture was poured into 1 ℓ o f ice water and the whole was stirred for more 30 minutes.

Deposited crystal was filtered, washed with water and 20.6 g of the desirable compound was obtained by drying. The yield was 62.0 %. Melting point 65-65.5°C.
¹H-NMR spectrum (CDCl3) δ ppm : 2.62 (SCH3, s)
MASS spectrum (Cl-Dl): 167, 120, 74

### (Example 1.)

### 2-(Nitroimino)imidazolidine:

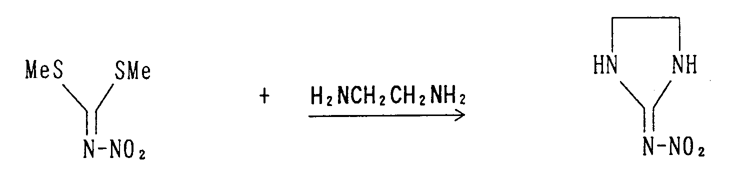

To a solution of 1.66 g (10 mmol) of dimethyl N-nit roimidodithiocarbonate in 5ml of chloroform, a solution of 0.72 g (12 mmol) of ethylenediamine in 1 ml of chloroform was added with stirring at 27 to 33°C under cooling by ice water.

The reaction mixture was stirred at room temperature for more 3 hours followed by evaporation of the solvent under reduced pressure. Deposited crude crystal was collected and washed by diethyl ether. 1.25 g of the desired crystalline compound was obtained by drying. The yield was 96.1 %. Melting point 213-215°C(decomposed).

### (Example 2. )

### 2-(Nitroimino)imidazoldine:

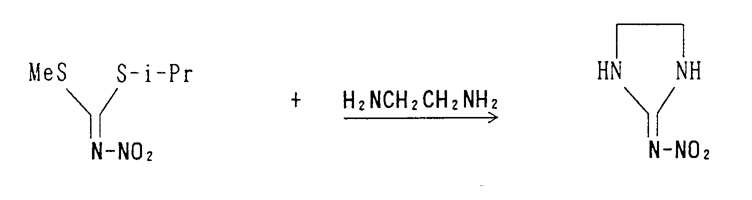

To a solution of 1.94 g (10 mmol) of S-isopropyl S'-methyl N-nitroim idodithiocarbonate in 5ml of chloroform, a solution of 0.66 g (11 mmol) of ethylenediamine in 1 ml of chloroform was added with stirring at 25 to 30°C under cooling by ice water.

The reaction mixture was stirred at room temperature for more 1.5 hours followed by the same treatment as Example 1. Thus, 1.20 g of the desired crystalline compound was obtained. The yield was 92.3 %.

### (Example 3.)

### 4-Methyl 2-(nitroimino)imidazolidine:

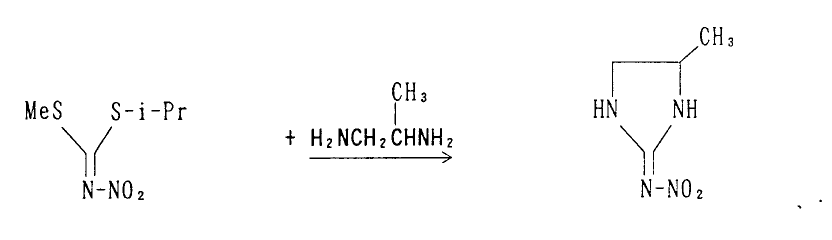

To a solution of 1.66 g (10 mmol) of dimethyl N-nit roimidodithiocarbonate in 5ml of chloroform, a solution of 0.81 g (11 mmol) of 1,2-propanediamine in 1 ml of chloroform was added with stirring at 28 to 38 °C u nder cooling by ice water.

The reaction mixture was stirred at room temperature for more one hour followed by the same treatment as Example 1. Thus, 1.29 g of the desired crystal line compound was obtained. The yield was 89.6 %.Melting point 172-3 °C.

### (Example 4.)

### 1-Methyl 2-(nitroimino)imidazolidine:

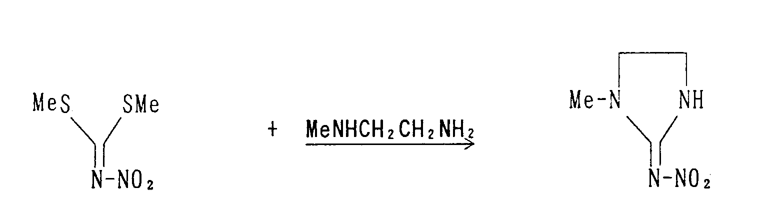

To a solution of 1.66 g (10 mmol) of dimethyl N-nitr oimidodithiocarbonate was dissolved and 0.82 g (11 mmol) of N-methylethylenediamine was added with stirring at 20 to 28 °C under cooling by ice water.

The reaction mixture was stirred at room temperature for more 3 hours followed by evaporating the solvent under reduced pressure. Crude crystal deposited was recrystallized from ethanol and dried. Thus 1.17 g of the desired compound was obtained. The yield was 81.3 %. Melting point 115-116.5 °C.

### (Example 5. )

### 1-(2-Chloro-5-pyridylmethyl)-2-(nitroimino)imidazolidine:

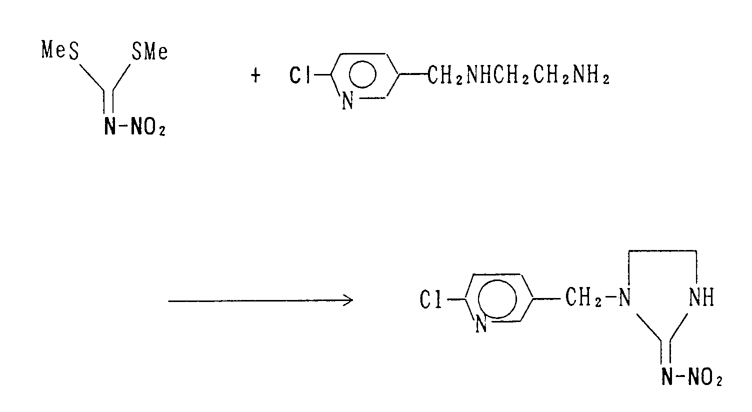

To a solution of 2.50 g (15 mmol) of dimethyl N-ni troimidodithiocarbonate in 10 ml of dichloromethane, 2.78 a (15 mmol) of N-(2-chloro-5-pyridylmethyl)ethylenediamine was added with stirring at 25 to 32°C under ice cooling .

The reaction mixture was stirred at room temperature for more 3 hours, followed by evaporating the solvent under reduced pressure. Crude crystal deposited was recrystallized from ethanol and dried. Thus, 3.07 g of the desired compound was obtained, The yield was 80.0 %. Melting point 134-6 °C.

### (Example 6.)

### 2-(Nitroimino)1,3-diazacyclohexane:

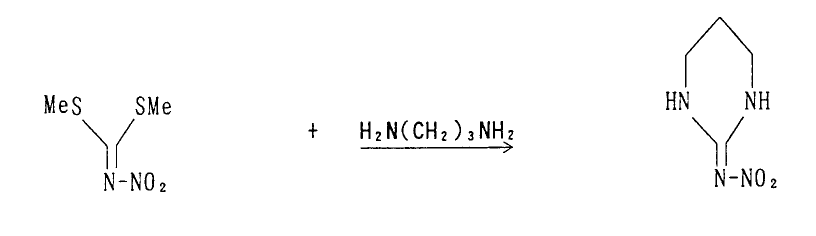

To a solution of 1.66 g (10 mmol) of dimethyl N-nitro imidodithiocarbonate in 5 ml of chloroform, 0.82 g (11 mmol) of trimethylenediamine was added with stirring at 25 to 37 °C under ice cooling.

After the reaction mixture was stirred at room temperature for more one hour, crude crystal deposited was filtered washed with chloroform and dried. Thus, 1.14 g of the desired compound was obtained. The yield was 79.2 %. Melting point 246-248°C (decomposed).

### (Example 7.)

### 2-(Nitroimino)1,3-diazacycloheptane:

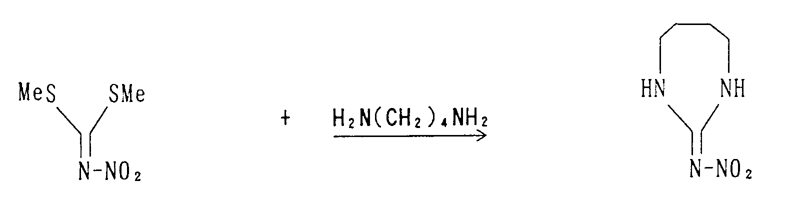

To a solution of 1.66 g (10 mmol) of dimethyl N-nit roimidodithiocarbonate in 5 ml of chloroform, 0.97 g (11 mmol) of tetramethylenediamine was added with stirring at 25 to 32 °C under ice cooling.

The reaction mixture was stirred at room temperature for more 1.5 hours, and treated as example 6. Thus, 1,42 g of the desired compound was obtained. The yield was 89.9 %. Melting point 182-4°C.

### (Comparative Example 1.)

### 2-(Nitroimino)imidazolidine:

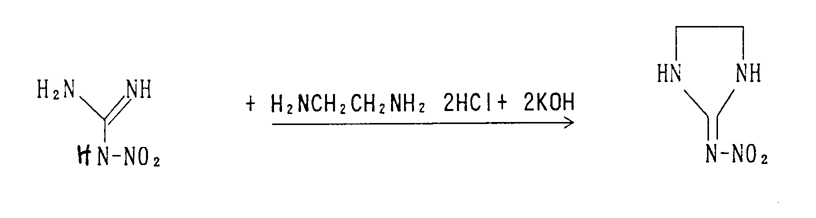

To a 50 ml of aqueous solution dissolving 22.4 g of potassium hydroxide (0.4 mol), 20.8 g (0.2 mol) of nitroguanidine and 26.6g (0. 2mol) of ethylenediamine di-hydrochloride were subsequently added with stirring at room temperature. The reaction mixture was heated to 65 to 70°C for 20 minutes and then cooled to 1°C. The deposited crystal was filtered and washed with small portion of cold water and dried. Thus, 9.6 g of the desired crystalline was obtained. The yield was 37 %.

### Industrial Applicability

Comparing the example 1 and 2 based on the preparation method in the present invention with comparative example 1, It is remarkable that yields of the desired nitrogen containing heterocycles are remarkably improved by the processes of the present invention.

A compound, for example, given in example 5 among the compounds prepared according to the invention is useful as an insecticide.

## Claims

1. A method of preparing nitrogen containing heterocyclic compounds represented by general formula [III] 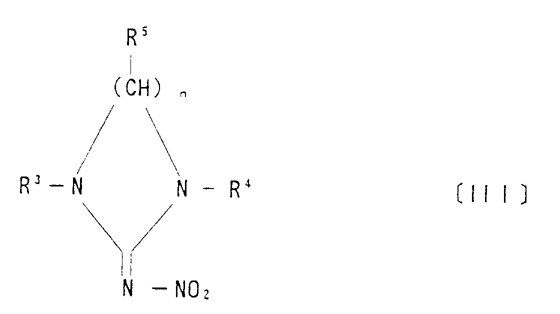 wherein R³ and R⁴ are hydrogen atom, an alkyl group or an alkyl group substituted by heterocycle respectively, R⁵ is hydrogen atom or an alkyl group and n is 2 to 4,
characterized in that esters of N-nitroimidodithiocarbonic acid represented by general formula [I] 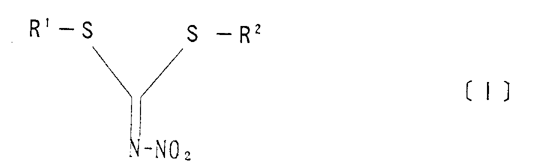 wherein R¹ and R² are an alkyl group respectively
and diamines represented by general formula [II] 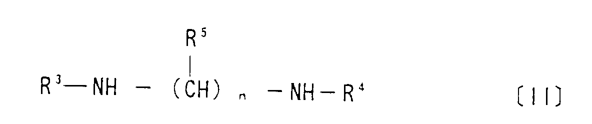 wherein R³, R⁴, R⁵ and n are same as defined above are reacted.

## Patentansprüche

1. Verfahren zur Herstellung von Stickstoff-haltigen heterocyclischen Verbindungen, die durch die allgemeine Formel (III) dargestellt werden 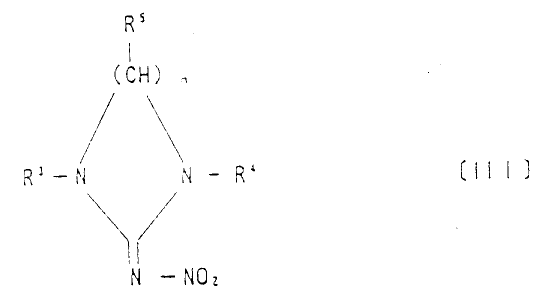 worin R³ und R⁴ jeweils ein Wasserstoffatom, eine Alkylgruppe oder eine durch einen Heterocyclus substituierte Alkylgruppe darstellen, R⁵ ein Wasserstoffatom oder eine Alkylgruppe bedeutet und n 2 bis 4 ist,
dadurch gekennzeichnet, daß Ester von N-Nitroimidodithiokohlensäure, die durch die allgemeine Formel (I) dargestellt werden 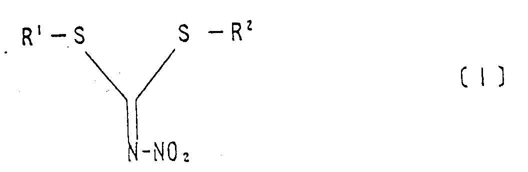 worin R¹ und R² jeweils eine Alkylgruppe darstellen,
und Diamine, die durch die allgemeine Formel (II) dargestellt werden 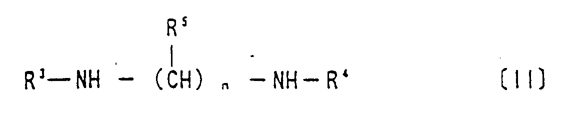 worin R³, R⁴, R⁵ und n wie oben definiert sind, umgesetzt werden.

## Revendications

1. Méthode de préparation de composés hétérocycliques contenant de l'azote représentés par la formule générale [III] 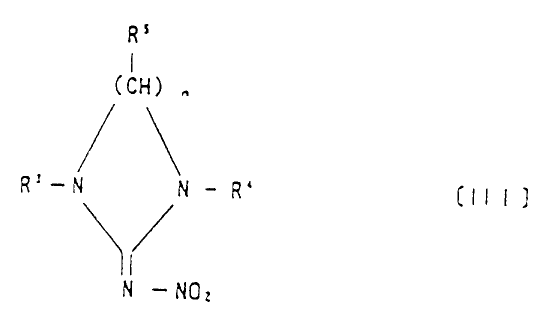 où R³ et R⁴ sont un atome d'hydrogène, un groupe alkyle ou un groupe alkyle substitué par un hétérocycle respectivement, R⁵ est un atome d'hydrogène ou un groupe alkyle et n est 2 à 4,
caractérisée en ce que l'on fait réagir des esters de l'acide N-nitroimidodithiocarbonique représentés par la formule générale [I] 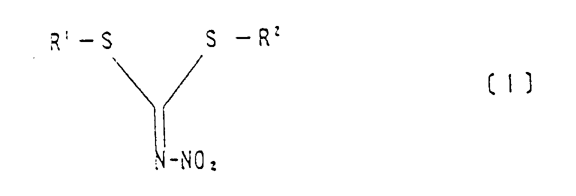 où R¹ et R² sont un groupe alkyle respectivement
et des diamines représentées par la formule générale [II] 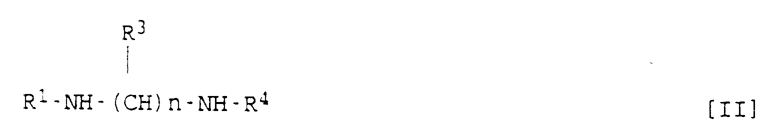 où R³, R⁴, R⁵ et n sont tels que définis ci-dessus.
